# EUROPEAN PATENT APPLICATION

(11) **EP 1 731 128 A1**
(43) Date of publication of application: **13.12.2006**
(21) Application number: 06115051.2
(22) Date of filing: 07.06.2006
(51) Int. Cl.: A61J 1/00, A61M 39/12, A61M 5/168

(54) **Closure device for containers or lines for administering medical or pharmaceutical fluids**

(30) Priority: 09.06.2005 IT MO20050141
(71) Applicant: ARIES S.r.l., 41037 Mirandola (Modena) (IT)
(72) Inventor: Greco, Francesco, 20030, Lentate sul Seveso MI (IT)
(74) Representative: Modiano, Micaela Nadia

(57) **Abstract**

A device (1) for closing containers or lines for administering medical or pharmaceutical fluids, comprising a first tubular element (3) and a second tubular element (4), which are open at the respective opposite ends and are mutually associated, at a respective end, so that they are mutually substantially coaxial and can move axially so as to slide and/or rotate with respect to each other, their respective free ends being respectively associable with the mouth of a container or an administration line of a fluid and with the mouth of a tank for feeding the fluid to the container, or to the administration line, or to the inlet of a fluid user device, and a valve element (16) for adjusting the flow of the fluid, which is formed inside the first and second tubular elements (3, 4), the sliding and/or relative rotation of the first and second tubular elements being adapted to move the valve element (16) from a closed configuration for blocking the flow of fluid to an open configuration for the inflow of the fluid from the tank to the container or to the administration line or from the container or from the administration line to the user device, and vice versa.

## Description

The present invention relates to a closure device for containers or lines for administering medical or pharmaceutical fluids, particularly syringes, pouches, catheters, tubes and the like.

Certain types of medical or pharmaceutical fluids, such as for example chemotherapy drugs and other drugs used to treat oncologic diseases, such as for example neoplasms and tumors, have a high degree of irritability or toxicity.

Usually, the individual doses provided for the therapy of the patients of the various wards of a health care center, for example a hospital, are prepared by specialized technicians who work in a pharmaceutical laboratory that serves the health care center.

The drugs are prepared in containers such as bags or bottles, from which the technicians, with the aid of conventional syringes, withdraw the individual doses to be administered. The syringes that contain the withdrawn doses are then taken to the individual wards, where health workers connect them to devices, such as drip-feed bags, lines, catheters or the like, for administration to the respective patients.

Both the laboratory technicians and the health workers must therefore pay attention during the various steps for the preparation, transport and administration of these drugs, so as to avoid accidental leaks or losses thereof; such leaks might in fact come into contact with the skin or eyes of the technicians or workers, causing irritations or intoxications.

Currently, in order to avoid accidental losses of fluid it is known to use caps or stoppers provided with a coupling of the Luer-Lok type to the complementary coupling formed on the syringes, bags and lines for administration.

Said caps or stoppers, however, are not free from drawbacks, including the fact that their use does not provide a safe solution to the danger of leaks of fluid from the syringes, containers or lines for administration during all the steps that constitute the withdrawal, transport and administration of fluid. In particular, for example, the danger of contamination occurs at the end of the step for withdrawal of the fluid, when the syringe, which contains the chosen dose, is disconnected from the supply bottle or pouch in order to be closed with the appropriately provided cap or stopper, and during the step for removing the stopper to connect the syringe to an administration device.

Leaks and dripping from the syringe are possible during these steps, with high risks of contamination for the worker or for the patient.

The aim of the present invention is to eliminate the drawbacks noted above of known stoppers and caps, by providing a closure device for containers or lines for administering medical or pharmaceutical fluids that allows to avoid accidental leaks or losses of medical or pharmaceutical fluids from the respective containers, in particular from syringes, during their filling, during their handling, for example for their transport within a health care center, and during the step for the administration of their contents to a patient.

Within this aim, an object of the present invention is to provide a closure device that ensures the safety of laboratory technicians and/or health workers assigned to the handling of containers of irritating or toxic medical or pharmaceutical fluids.

Another object of the present invention is to provide a closure device that ensures the sterility of the medical and pharmaceutical fluids contained in the containers or in the administration lines.

Another object of the present invention is to provide a closure device that is simple, relatively easy to provide in practice, safe in use, effective in operation, and has a relatively low cost.

This aim and these and other objects that will become better apparent hereinafter, are achieved by a device for closing containers or lines for administering medical or pharmaceutical fluids, characterized in that it comprises a first tubular element and a second tubular element, which are open at the respective opposite ends and are mutually associated, at a respective end, so that they are mutually substantially coaxial and can move axially so as to slide and/or rotate with respect to each other, their respective free ends being respectively associable with the mouth of a container or an administration line of a fluid and with the mouth of a tank for feeding the fluid to the container, or to the administration line, or to the inlet of a fluid user device, and a valve element for adjusting the flow of the fluid, which is formed inside the first and second tubular elements, the sliding and/or relative rotation of the first and second tubular elements being adapted to move the valve element from a closed configuration for blocking the flow of fluid to an open configuration for the inflow of the fluid from the tank to the container or to the administration line or from the container or from the administration line to the user device, and vice versa.

Further characteristics and advantages of the present invention will become better apparent from the following detailed description of two preferred but not exclusive embodiments of a device for closing containers or lines for administering medical or pharmaceutical fluids, illustrated by way of non-limiting example in the accompanying drawings, wherein:

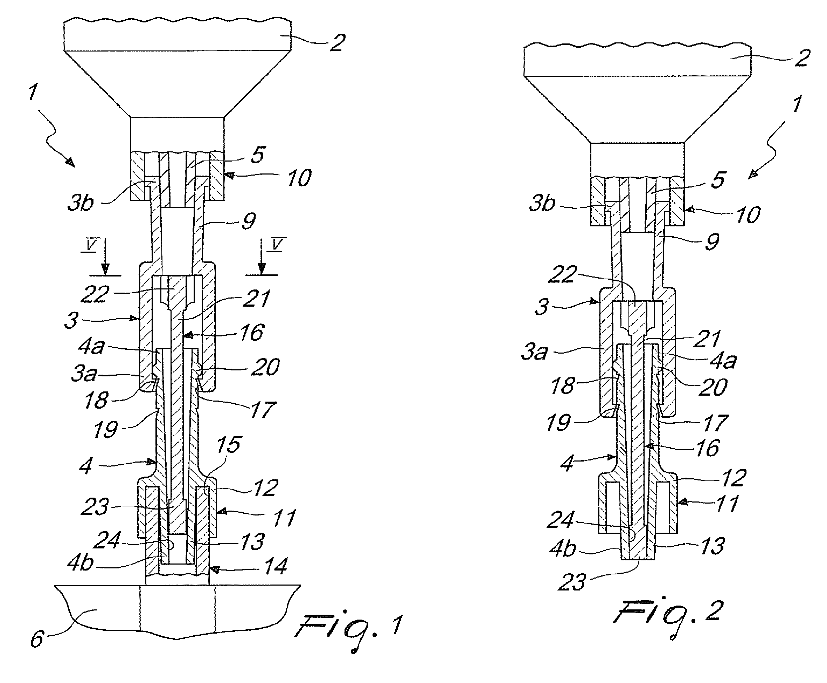

Figure 1 is a sectional view of a first embodiment of the closure device according to the invention in the open configuration;
Figure 2 is a sectional view of the closure device of Figure 1 in the closed configuration;

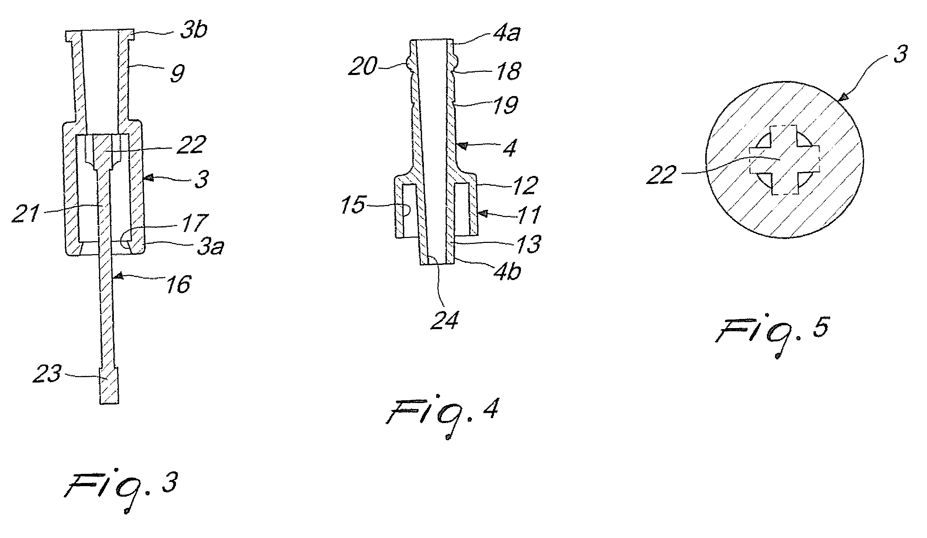

Figures 3 and 4 are sectional views respectively of a first element and of a second element that constitute the device according to the preceding figures;

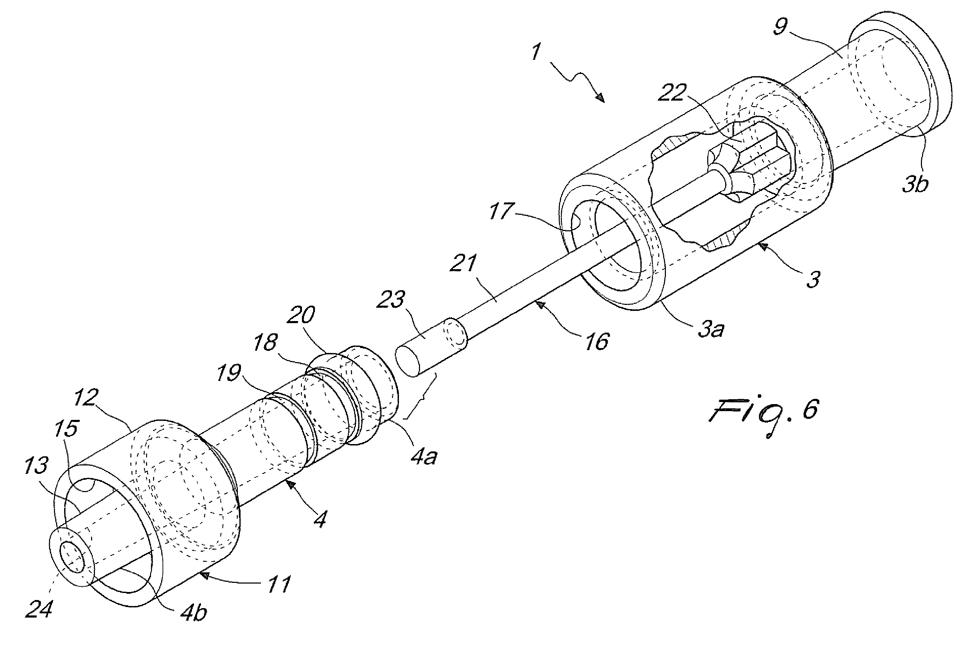

Figure 5 is a sectional view, taken along the line V-V of Figure 1, of the closure device according to the invention;
Figure 6 is an exploded axonometric view of the closure device of the preceding figures;
Figures 7-11 illustrate a syringe to which the closure device of the preceding figures is applied, in various steps of its use: during the withdrawal of a dose of fluid from a supply tank, during transport and during the transfer of the fluid from the syringe to a user device, such as a device for administration to a patient;
Figure 12 is an axonometric exploded view of a second embodiment of the closure device according to the invention.

With reference to the figures, the reference numeral 1 generally designates a device for closing containers or lines for administering medical or pharmaceutical fluids.

The device 1 can be applied to a container for fluids in general, in particular to a syringe 2, to which reference is made hereinafter without thereby limiting the present invention. As an alternative, the device can be applied to administration lines or to other containers, such as pouches, catheters and the like.

The device 1 comprises a first tubular element 3 and a second tubular element 4, which are open at their respective opposite ends.

The first tubular element 3 and the second tubular element 4 are mutually associated at a respective end 3a and 4a, and are mutually coaxial and axially movable so as to slide and/or rotate with respect to each other.

In particular, the first tubular element 3 and the second tubular element 4 are coupled together so that they can slide axially with a slack cup-like coupling, in which the end 4a of the second tubular element 4 is inserted in the end 3 a of the first tubular element 3.

The first tubular element 3 comprises a free end 3b, which is arranged opposite the end 3a associated with the second tubular element 4 and is associable with the tip 5 of the syringe 2.

The second tubular element 4 comprises a free end 4b, which can be associated with the mouth of a tank 6 for supplying the fluid or with the inlet of a fluid user device, such as for example a device for administering the fluid to a patient, such as a pouch 7 or a catheter 8.

The free ends 3b and 4b respectively of the first and second tubular elements 3 and 4 are provided with a respective connector of the male or female Luer-Lok type. In particular, the free end 3b is provided with a female Luer-Lok connector 9, which is frustum-shaped and expands outward and can be inserted partially in a male Luer-Lok connector 10 with which the syringe 2 is provided.

The free end 4b is provided with a connector of the male Luer-Lok type 11, which is constituted by an outer cylindrical jacket 12 and by a connecting element 13, which is arranged inside the cylindrical jacket 12, is coaxial thereto and is shaped like a frustum which converges toward the outside.

The tank 6 that contains the fluid, or the generic fluid user device, such as the pouch 7 or the catheter 8, are provided with a female Luer-Lok connector 14, to which the male Luer-Lok connector 11 can be fixed.

In particular, the wall of the female Luer-Lok connector 14 can be inserted within an interspace 15 delimited by the internal surface of the cylindrical jacket 12 and by the external surface of the connecting element 13.

The device 1 further comprises a valve element 16 for adjusting the flow of the fluid, which is formed inside the first and second tubular elements 3 and 4 in a coupled configuration.

The valve element 16 can be activated, by means of the sliding and/or relative rotation of the first tubular element 3 and of the second tubular element 4, for transition from a closed configuration for blocking the flow of fluid to an open configuration for the inflow of the fluid, from the tank 6 to the container, i.e., to the syringe 2, or from the container, i.e., from the syringe 2 to the user device, i.e., to the pouch 7 or to the catheter 8, and vice versa.

The relative sliding between the first and second tubular elements 3 and 4 and specifically the transition from the open configuration to the closed configuration or vice versa of the valve element 16 is controlled by retention means, which are formed between the first and second tubular elements 3 and 4.

Said retention means allow to limit the stroke of the relative sliding between the first and second tubular elements 3 and 4 and comprise a retention tooth 17, which protrudes toward the center on the internal surface of the first tubular element 3, advantageously at the inlet for the insertion of the second tubular element 4 formed at its end 3 a.

The retention tooth 17 is adapted to engage a first annular groove 18 and a second annular groove 19, which are formed and mutually spaced on the outer surface of the second tubular element 3.

Conveniently, the tooth can flex elastically in order to allow the transition from the closed configuration to the open configuration and vice versa.

The distance between the first groove 18 and the second groove 19 determines the breadth of the stroke of the relative sliding between the first and second tubular elements 3 and 4.

Further, the second tubular element 4 has, at the internal surface of its end 4a coupled to the first tubular element 3, an annular collar 20, which forms a sealing ring for the fluid with the internal surface of the corresponding end 3 a of the first tubular element 3.

The annular collar 20 therefore performs a sealing function, adapted to prevent the escape of the fluid proximate to the connected ends 3a and 4a of the first and second tubular elements 3 and 4 during the various steps of the use of the syringe 2, i.e., during the withdrawal of the fluid from a tank 6, during transport and during transfer of the fluid to a user device, a pouch 7 or a catheter 8.

The valve element 16 comprises a flow control element, which comprises a stem 21 which is accommodated within the first and second tubular elements 3 and 4 in a coupled configuration and substantially coaxially thereto.

An actuation end 22 of the stem 21 is rigidly associated with one of the first and second tubular elements 3 and 4, while the opposite end is provided with a head 23, which can be inserted hermetically in a corresponding sealing seat 24 formed within the other of the first and second tubular elements 3 and 4.

In particular, the actuation end 22 is fixed to the first tubular element 3 proximate to its free end 3b, and the stem 21 lies inside the second tubular element 4.

The head 23 is provided at the end of the stem 21 that lies opposite the actuation end 22 and can engage the sealing seat 24 formed inside the second tubular element 4 and proximate to its free end 4b.

In particular, the actuation end 22 is radially shaped, preferably cross-shaped, so as to leave free openings for the flow of the fluid in both directions of flow, i.e., in input and in output with respect to the first tubular element 3 and therefore with respect to the syringe 2.

The relative sliding between the first and second tubular elements 3 and 4 in the direction for mutual approach moves the valve element 16 from the open configuration to the closed configuration; in this configuration, the retention tooth 17 engages the second groove 19 and the head 23 engages the sealing seat 24, closing hermetically the opening of the free end 4b of the second tubular element 4.

Advantageously, the sealing seat 24 is shaped like a frustum which converges toward the outside of the free end 4b of the second tubular element 4 and corresponds to the internal surface of the connecting element 13 of the male Luer-Lok connector 11.

The head 23 is likewise frustum-shaped.

Alternative and equivalent embodiments of the valve element 16 and/or of the flow control element are also possible.

For example, in the flow control element the stem 21 might be rigidly coupled to the second tubular element 4 and the head 23 might engage hermetically a seat formed in the first tubular element 3.

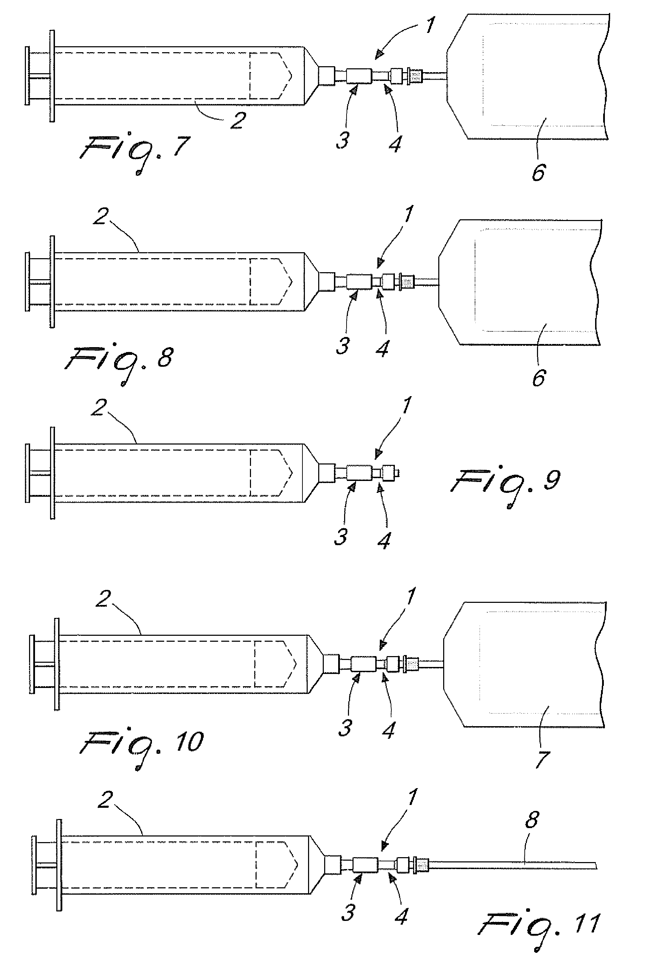

The operation of the present invention, as shown in Figures 7 to 11, is as follows.

The closure device 1 is connected to a container for the fluid, in particular to the tip 5 of the syringe 2. Connection occurs by mating the female Luer-Lok connector 9 at the end 3b of the first tubular element 3 with the male Luer-Lok connector 10 of the syringe 2.

As shown in Figure 7, the device 1 is connected, at the end 4b of the tubular element 4, to the tank 6 for supplying the fluid in order to prepare a single dose. The connection is provided by means of the connection between the male Luer-Lok connector 11 at the end 4b and the female Luer-Lok connector 14 provided on the tank 6.

For the step for the withdrawal of the fluid from the fluid supply tank 6, the valve element 16 of the device 1 must be in the open inflow configuration before connection to the tank 6 or after its connection thereto.

The transition from the closed flow blocking configuration to the open inflow configuration of the valve element 16 is the result of the relative sliding, in mutually opposite directions, between the first tubular element 3 and the second tubular element 4, positioning the retention tooth 17 at the first annular groove 18.

In this open configuration, the head 23 of the valve element 16 is disengaged from the sealing seat 24 and an opening is formed between the two for the passage of the fluid from the fluid supply tank 6, through the second tubular element 4 and through the first tubular element 3 and at the openings formed by the actuation end 22, to the syringe 2.

Once the withdrawal has occurred by means of a pulling action applied to the plunger of the syringe 2, and therefore once a given dose of fluid has been withdrawn from the tank 6, the valve element 16 is moved to the closed configuration for blocking the flow.

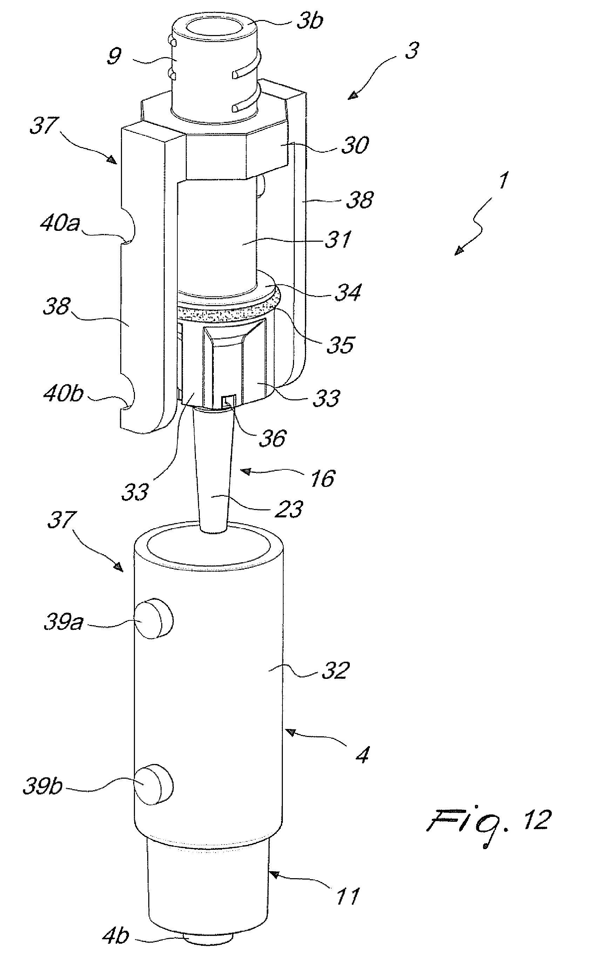

As shown in Figure 8, this occurs by mutual sliding toward each other of the first tubular element 3 and the second tubular element 4, with positioning of the retention tooth 17 at the second annular groove 19.

In this closed configuration, the head 23 is engaged on the surface of the sealing seat 24 for a hermetic closure of the opening on the free end 4b of the second tubular element 4.

As shown in Figure 8, the male Luer-Lok connector 11, at the end 4b, is removed from the tank 6, and the syringe 2, which comprises the closure device 1 applied to its tip 5, is carried to the destination ward for administration.

The syringe 2 containing the fluid is applied by means of the device 1 to an administration device. In particular, the device 1 can be connected by means of the male Luer-Lok connector 11 to a female Luer-Lok connector of a pouch 7 for administration or of a catheter 8 applied to a patient, as shown respectively in Figures 10 and 11.

Once the device 1 has been connected, the valve element is brought to the open configuration by way of the relative sliding, in mutually opposite directions, of the first tubular element 3 and the second tubular element 4, and the fluid is introduced, by applying pressure to the plunger of the syringe 2, in the administration pouch 7 or along the catheter 8.

Once the fluid has been administered, before removing the syringe 2 with the device 1 from the pouch 7 or from the catheter 8, the valve element 16 is moved to the closed configuration, so as to avoid leaks of residues or excess fluid still present inside the syringe 2 or inside the first and second tubular elements 3 and 4.

In an alternative embodiment, shown in Figure 12, the first tubular element 3 is constituted substantially by a first portion, which is arranged at the free end 3b and in which the connector of the female Luer-Lok type 9 is formed; by a second intermediate portion 30, which has a larger cross-section and is preferably annular; and a third portion, which is constituted by a cylindrical jacket 31 and forms the end 3a, said portions being blended together.

The second tubular element 4 instead comprises a tubular segment 32, which is blended with the connector of the male Luer-Lok type 11 on the side directed toward the first tubular element 3, which is adapted to form the free end 4a. The first and second tubular elements, respectively 3 and 4, are therefore coupled at the respective free ends 3a and 4a with a coupling of the loose cup-shaped type, in which the cylindrical jacket 31 is accommodated so that it can slide within the tubular segment 32.

The actuation end 22 of the stem 21, not shown in the figure, is rigidly associated inside the cylindrical jacket 31 and its conical head 23 protrudes from the end 3 a.

The free end of the cylindrical jacket 31 is shaped so as to form a plurality of radial ribs 23, which are connected to a circumferential protrusion 34, in the lateral spine of which a sealing ring 35 is accommodated which is adapted to interfere with the inside wall of the tubular segment 32 in order to avoid leaks of fluid, said ring constituting a part of the retention means.

In this particular embodiment, said retention means in fact have a first annular groove and a second annular groove formed on the internal wall of the tubular segment 32, not shown in Figure 12, which are conveniently spaced by an extent that corresponds to the relative stroke between the first and second tubular elements, respectively 3 and 4, in order to pass from the open configuration to the closed configuration and vice versa. In these end configurations, the sealing ring 35 engages alternatively one of said annular grooves, limiting the relative sliding between the tubular elements 3 and 4.

At the free end of the cylindrical jacket 31 there are a plurality of recesses 36, which are spaced with the radial ribs 33 and are delimited in a downward region by the head 23.

During withdrawal, with the device 1 in the open configuration, the fluid passes through the recesses 36 and flows within the first tubular element 3 until it exits from the end 3b.

Conveniently, in addition to the retention means described above, there are means 37 for temporarily locking the device 1 in the open and closed configuration, which can be activated by the operator and are interposed between the first and second tubular elements, respectively 3 and 4.

The temporary locking means 37 comprise at least one and more preferably two wings 38, which protrude from the ring 30 and are directed toward the second tubular element 4 and are aligned with it, and between which the cylindrical jacket 31 is arranged. The temporary locking means 37 further comprise at least two pins 39, which protrude in a radial direction from the outer surface of the tubular segment 32 and are aligned along its longitudinal direction. In the figure, the reference numeral 39a designates the pin arranged proximate to the end 4a and the reference numeral 39b designates the pin that is arranged furthest from said end. The relative distance between the pins 39 is equal to the relative stroke between the tubular elements 3 and 4 for passing from the open configuration to the closed configuration and vice versa. In the figure, the pins 39 have a circular cross-section, but they might also have a different configuration. Two notches 40 are formed on each of the wings 38 and are each suitable to accommodate a corresponding pin 39. In the figure, the reference numeral 40a designates the notch arranged proximate to the ring 30 and the reference numeral 40b designates the notch that is arranged furthest therefrom.

Conveniently, in the open configuration, during withdrawal, the operator can produce a relative rotation between the first and second tubular elements, respectively 3 and 4, by acting on the ring 30 until the pin 39a engages in one of the notches 40b of one of the two wings 38. In the closed configuration, instead, the operator can produce a relative rotation between the first and second tubular elements, respectively 3 and 4, by acting on the ring 30 until the pins 39a and 39b engage in the notches 40a and 40b respectively of one of the two wings 38.

In order to disengage the pins 39 from the corresponding notches 40 it is then sufficient to produce the relative rotation of the tubular elements 3 and 4 in the opposite direction.

In practice it has been found that the described invention achieves the proposed aim and objects, and in particular the fact is stressed that it allows to preserve the sterility of medical or pharmaceutical fluids, avoiding accidental losses or leaks from the respective container or lines for administration, during the filling step, during their handling, for example for transport inside a health care center, and during the administration of their contents to a patient.

The invention thus conceived is susceptible of numerous modifications and variations, all of which are within the scope of the appended claims.

All the details may further be replaced with other technically equivalent elements.

In practice, the materials used, as well as the contingent shapes and dimensions, may be any according to requirements without thereby abandoning the scope of the protection of the appended claims.

The disclosures in Italian Patent Application No. MO2005A000141, from which this application claims priority, are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A device for closing containers or lines for administering medical or pharmaceutical fluids, **characterized in that** it comprises a first tubular element and a second tubular element, which are open at the respective opposite ends and are mutually associated, at a respective end, so that they are mutually substantially coaxial and can move axially so as to slide and/or rotate with respect to each other, their respective free ends being respectively associable with the mouth of a container or an administration line of a fluid and with the mouth of a tank for feeding said fluid to said container, or to said administration line, or to the inlet of a fluid user device, and a valve element for adjusting the flow of said fluid, which is formed inside said first and second tubular element, the sliding and/or relative rotation of said first and second tubular elements being adapted to move said valve element from a closed configuration for blocking the flow of said fluid to an open configuration for the inflow of said fluid from the tank to said container or to said administration line or from said container or from said administration line to said user device, and vice versa.

2. The device according to claim 1, **characterized in that** said free end of said first tubular element and/or said free end of said second tubular element comprise a connecting element of the female or male Luer-Lok type.

3. The device according to one or more of the preceding claims, **characterized in that** said first and second tubular elements are mutually coupled with a loose cup-type coupling.

4. The device according to one or more of the preceding claims, **characterized in that** said first and second tubular elements are mutually coupled so that they can slide axially.

5. The device according to one or more of the preceding claims, **characterized in that** between said first and second tubular elements there are retention means adapted to limit the stroke of the relative mutual sliding of said first and second tubular elements that is adapted to move said valve element between said closed configuration and said open configuration and vice versa.

6. The device according to one or more of the preceding claims, **characterized in that** said retention means comprise a retention tooth, which is formed so as to protrude toward the center on the internal surface of said first tubular element and is adapted to engage alternatively with a first or second annular groove, which are mutually spaced on the outer surface of said second tubular element.

7. The device according to one or more of the preceding claims, **characterized in that** said second tubular element has, at the internal surface of its end coupled to said first tubular element, an annular collar, which forms a sealing ring with the internal surface of the corresponding end of said first tubular element.

8. The device according to one or more of claims 1 to 5, **characterized in that** said retention means comprise a sealing ring, which protrudes from the outer surface of said first tubular element and is adapted to engage alternately a first annular groove or a second annular groove mutually spaced on the internal surface of said second tubular element.

9. The device according to one or more of the preceding claims, **characterized in that** said valve element comprises a flow control element, which comprises a stem which is accommodated substantially coaxially within said first and second tubular elements in a coupled configuration and has an actuation end which is rigidly associated with one of said first and second tubular elements and the opposite end provided with a head which can be inserted hermetically in a corresponding sealing seat formed within the other of said first and second tubular elements.

10. The device according to one or more of the preceding claims, **characterized in that** said actuation end is associated proximate to said free end of said first tubular element, said stem protruding inside said second tubular element and said sealing seat being formed at the free end of said second tubular element.

11. The device according to one or more of the preceding claims, **characterized in that** said actuation end has a radial shape.

12. The device according to one or more of the preceding claims, **characterized in that** said sealing seat is frustum-shaped and converges toward the outside of the free end of said second tubular element.

13. The device according to one or more of the preceding claims, **characterized in that** said head is frustum-shaped.

14. The device according to one or more of the preceding claims, **characterized in that** it comprises means for temporary locking in the open and/or closed configuration, which are interposed between said first and second tubular elements and can be actuated by an operator by means of a relative rotation of said tubular elements.

15. The device according to claim 14, **characterized in that** said temporary locking means comprise at least one wing, which protrudes from said first tubular element, and at least one pin, which protrudes from the outer surface of said second tubular element, at least one notch being provided on said wing for accommodating said pin.

16. The device according to claim 15, **characterized in that** said locking means comprise two of said pins, aligned along said second tubular element, said wing being provided with two of said notches which are each adapted to accommodate one of said pins.
